# EUROPEAN PATENT APPLICATION

(11) **EP 3 705 160 A1**
(43) Date of publication of application: **09.09.2020**
(21) Application number: 18873183.0
(22) Date of filing: 15.10.2018
(51) Int. Cl.: A61N 5/06

(54) **FACE-WEARABLE RHINITIS TREATMENT DEVICE USING LIGHT THERAPY**

(30) Priority: 01.11.2017 KR 20170144844; 01.11.2017 KR 20180014378
(71) Applicant: Double H Ltd., Seongnam-si, Gyeonggi-do 13488 (KR)
(72) Inventor: LEE, Kyung Han, Yongin-si Gyeonggi-do 16909 (KR)
(74) Representative: dompatent von Kreisler Selting Werner - Partnerschaft von Patent- und Rechtsanwälten mbB
(86) International application number: PCT/KR2018/012132
(87) International publication number: WO 2019/088502

(57) **Abstract**

Provided is a face-wearing type rhinitis treatment device using light therapy. The face-wearing type rhinitis treatment device includes a light irradiating unit configured to irradiate light for rhinitis treatment into the nose of a patient; and a wearing main body, which is accommodated at positions spaced below the eyes of the patient and is wearable on the face of the patient, The face-wearing type rhinitis treatment device according to present invention may stably maintain the light irradiating unit for treating rhinitis at a correct position under the nose to improve treatment effect and may minimize restrictions of movement of the patient after wearing the face-wearing type rhinitis treatment device to minimize discomfort of the patient.

## Description

### TECHNICAL FIELD

The present invention relates to a face-wearing type rhinitis treatment device using light therapy, and more particularly, to a face-wearing type rhinitis treatment device using light therapy that a patient may comfortably wear and is capable of stably irradiating light into a nose.

### BACKGROUND ART

Recently, the number of patients suffering from rhinitis has been increasing.

In general, rhinitis refers to inflammation of nasal mucosa inside the nose, which is a respiratory organ of the human body, and includes acute and chronic rhinitis, viral rhinitis caused by a cold, and allergic rhinitis.

Infection with rhinitis not only makes it difficult to breathe, but also causes headaches and distractions, leading to greater discomfort. Rhinitis may be dangerous to children including infants by causing difficulty breathing.

Particularly, in recent years, many patients suffer from rhinitis disease due to the increase of environmental pollution and air pollutants.

As a treatment for rhinitis, drug therapy using anti-histamine, topical steroids, anti-leukotriene, anti-choline spray, etc. is mainly used. However, recently, light treatment techniques using visible rays and infrared rays have been actively used.

Therefore, a face-wearing type rhinitis treatment device using light therapy for treating rhinitis by using visible rays or infrared rays has been proposed.

A conventional face-wearing type rhinitis treatment device using light therapy, which uses light to treat rhinitis, inserts a lamp that emits light for treatment into the nostrils and irradiates light from the entrances of the nostrils into the nose and, by irradiating the light to the skin inside the nose, enhances blood flow and soothes symptoms.

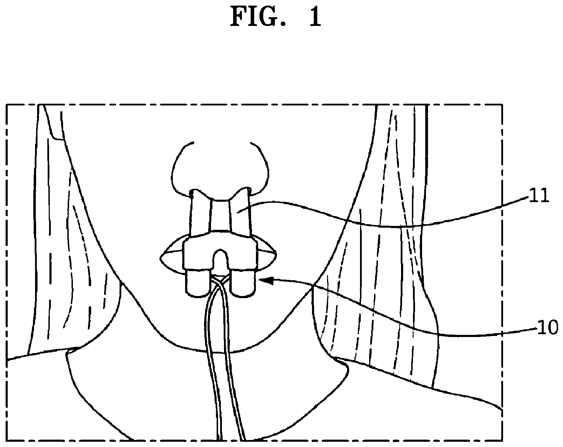

FIG. 1 is a schematic diagram showing a comparative example of a face-wearing type rhinitis treatment device using light therapy for treating rhinitis using light.

Referring to FIG. 1, a face-wearing type rhinitis treatment device 10 according to the comparative example includes a pair of rhinitis treatment light-emitting protrusions 11 provided with lamps that emit light for treating rhinitis, such as visible rays and infrared rays, at an end thereof and performs treatment by inserting the pair of rhinitis treatment light-emitting protrusions 11 into both nostrils of a patient.

However, the face-wearing type rhinitis treatment device 10 simply inserts the pair of rhinitis treatment light-emitting protrusions 11 into both nostrils of the patient, and thus, the pair of rhinitis treatment light-emitting protrusions 11 may be easily separated from the nostrils of the patient during treatment. In other words, the face-wearing type rhinitis treatment device 10 according to the comparative example needs to restrict the movement of the patient during rhinitis treatment, and the patient needs to be careful, such that the pair of rhinitis treatment light-emitting protrusions 11 are not separated from the nostrils.

Also, when the pair of rhinitis treatment light-emitting protrusions 11 are separated from the nostrils during the operation of the face-wearing type rhinitis treatment device 10, it is difficult to continue rhinitis treatment, and thus, it is difficult to obtain a desired treatment effect. Also, since the pair of rhinitis treatment light-emitting protrusions 11 may not be stably maintained at correct positions inside the nostrils, the rhinitis treatment effect is deteriorated.

Korean patent registration no. 0987729, which is 'Rhinitis treatment device using an LED' (registered on October 10, 2010), has been proposed as a patent related to the present invention.

Korean patent registration No. 0987729, which is 'Rhinitis treatment device using an LED' (2010.10.07.registration), discloses a fixing member that is connected to a main body having an LED light source and is worn on a part of the head of a patient to support the main body, wherein the fixing member is provided as eyeglasses that may be hung on and fixed to the ears of the patient.

However, the fixing member of Korean patent registration No. 0987729, which is 'Rhinitis treatment device using an LED' (registered on October 10, 2010), has an eyeglasses-like form, that is, a structure that includes legs that are hung on the ears and a nose support that is supported on the nose, and thus the weight of the rhinitis treatment device concentrates on the nose and causes discomfort.

In other words, it is difficult for a patient to wear the rhinitis treatment device disclosed in Korean patent registration No. 0987729, which is 'Rhinitis treatment device using an LED' (registered on October 07, 2010), for a long time, because the weight thereof concentrates on the nose of the patient. Also, there are movement restrictions of a main body due to external impact or movement of the patient, and the main body may be moved due to external impact or movement of the patient during a treatment. As a result, the main body may not be stably maintained at correct positions inside the nostrils, and thus, a rhinitis treatment effect is deteriorated.

### DESCRIPTION OF EMBODIMENTS

### TECHNICAL PROBLEM

The present invention provides a face-wearing type rhinitis treatment device using light therapy that a patient may comfortably wear and stably irradiates light into the nose to improve a rhinitis treatment effect.

### SOLUTION TO PROBLEM

According to an aspect of the present invention, there is provided a face-wearing type rhinitis treatment device including a light irradiating unit configured to irradiate light for rhinitis treatment into the nose of a patient; and a wearing main body, which is accommodated at positions spaced below the eyes of the patient and is wearable on the face of the patient.

The wearing main body may include a nose insertion groove, which is accommodated on the nose of the patient as the nose of the patient is inserted thereto; a face accommodating portion accommodated on the face of the patient at positions spaced below the eyes of the patient; and a nose supporter, which is arranged at a leading end portion of the nose insertion groove for the light irradiating unit to protrude to the rear side of the nose supporter, protrudes from the lower surface of the wearing main body, and is formed to partially cover an opened lower portion of the nose insertion groove, such that an end of the nose of the patient between the nostrils is inserted thereto.

The wearing main body may be supported as lower portions of both side surfaces of the nose insertion groove are accommodated on a nose wing on the both sides of the nose.

The wearing main body may further include a nasal bone accommodating portion, which includes a boundary edge which connects a rear surface of the face accommodating portion to inner surfaces of the nose insertion groove and is formed round to be accommodated on a nasal bone of the patient.

The wearing main body may further include a cheekbone accommodating portion arranged to protrude from side ends of the face accommodating portion to be placed on a cheekbone of the patient.

The face accommodating portion has a rearward convex shape, and at least a portion of a rearward convex portion may be accommodated above the eyelids defining the eye sockets of the patient.

The nasal bone accommodating portion includes a round lower portion.

In the wearing main body, an edge portion at the boundary between the inner surface of the cheekbone accommodating portion and the face accommodating portion is round.

The face-wearing type rhinitis treatment device may further include a control unit configured to control an operation of the light irradiating unit; and an on/off switch, which is arranged on a top surface of the wearing main body and turns the light irradiating unit on or off.

The face-wearing type rhinitis treatment device may further include wearing support legs, which are arranged on both sides of the wearing main body and are located on both sides of the head of the patient.

The face-wearing type rhinitis treatment device may further include an edge frame member, which surrounds a front surface and both side surfaces of the wearing main body and is rotatably hinge-connected to the wearing support legs at both ends.

The face-wearing type rhinitis treatment device may further include ear hooks, which are arranged at rear ends of the wearing support legs to be hung on the ears of the patient and are movable in a lengthwise direction of the wearing support legs.

The wearing support legs may further include moving guide rails, which are formed as slits with opened side surfaces and connect the ear hooks to the wearing support legs to be movable in the lengthwise direction of the wearing support legs.

Each of the ear hooks may include an ear hook body overlapping with the wearing support leg and moving in the lengthwise direction of the wearing support leg; and an ear hook support, which extends from the rear end of the ear hook body to protrude downward and is hung on the ear of the patient.

Each of the ear hooks may include a moving body, which penetrates through and is coupled with the moving guide rail and is supported above and below the moving guide rail to move in a lengthwise direction of the moving guide rail; and a moving guide body, which has a size larger than that of the moving body and, is located at an end of the moving body and an outer side surface of the wearing support leg to move while being supported by the outer side surface.

The moving guide rail is located at the outer side surface of the wearing support leg, and the wearing support leg may include a first moving rail groove to which the moving guide body is inserted to guide movement of the moving guide body while the first moving rail groove supports the upper and lower portions of the moving guide body.

The moving guide rail is located at the inner side surface of the wearing support leg, and the wearing support leg may include a second moving rail groove having an opened bottom to support the upper portion of the ear hook and guide movement of the moving guide body.

Each of the ear hook supports may include a moving guide protrusion, which is formed to protrude from the ear hook body and supports the lower portion of the wearing support leg to move in close contact with the wearing support leg.

### ADVANTAGEOUS EFFECTS OF DISCLOSURE

According to the present invention, the weight of a rhinitis treatment device is distributed to and supported by the nose and portions above the eyes of a patient to provide comfort while wearing the rhinitis treatment device, thereby minimizing discomfort of the patient during treatment and enabling stable long-time rhinitis treatment. As a result, satisfaction and treatment effect may be improved simultaneously.

The present invention prevents movement of the light irradiating unit for treating rhinitis caused by external factors after wearing a rhinitis treatment device, and thus the light irradiating unit for treating rhinitis may be stably maintained at a correct position under the nose to further improve treatment effect and reduce treatment time. Also, restrictions of movement of the patient after wearing the rhinitis treatment device may be minimized to minimize discomfort of the patient.

### BRIEF DESCRIPTION OF DRAWINGS

FIG. 1 is a schematic diagram showing a comparative example of a face-wearing type rhinitis treatment device using light therapy for treating rhinitis using light.
FIG. 2 is a perspective view of a face-wearing type rhinitis treatment device using light therapy, according to an embodiment of the present invention.
FIG. 3 is a bottom perspective view of a face-wearing type rhinitis treatment device using light therapy, according to an embodiment of the present invention.
FIG. 4 is a top view of a face-wearing type rhinitis treatment device using light therapy, according to an embodiment of the present invention.
FIG. 5 is a lateral view of a face-wearing type rhinitis treatment device using light therapy, according to an embodiment of the present invention.
FIG. 6 is a cross-sectional view taken along a line A-A' of FIG. 5.
FIG. 7 is a use state diagram showing an example of wearing a face-wearing type rhinitis treatment device using light therapy, according to the present invention.

### MODE OF DISCLOSURE

Hereinafter, embodiments of the present invention will be described with reference to the accompanying drawings. The present invention will be described in detail by focusing on the parts necessary to understand the operation and action according to the present invention. In describing the embodiments of the present invention, descriptions of technical features that are well known in the art to which the present invention belongs and are not directly related to the present invention will be omitted. This is to more clearly describe the present invention by omitting unnecessary description.

Also, in describing the components of the present invention, different reference numerals may be given to components having the same names according to the drawings, and the same reference numerals may be given to different drawings. However, even in such a case, it does not mean that the corresponding components have different functions according to embodiments or does not mean that they have the same functions in different embodiments, and the function of each component should be determined based on the description of the each component in corresponding embodiments.

As used herein, the singular forms "a", "an" and "the" mean one or more of the elements described interchangeably with the term "at least one".

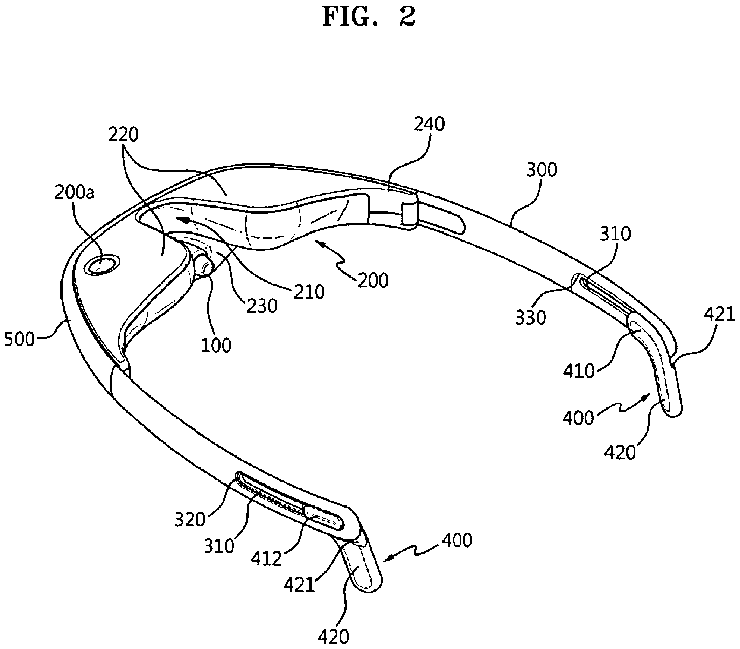

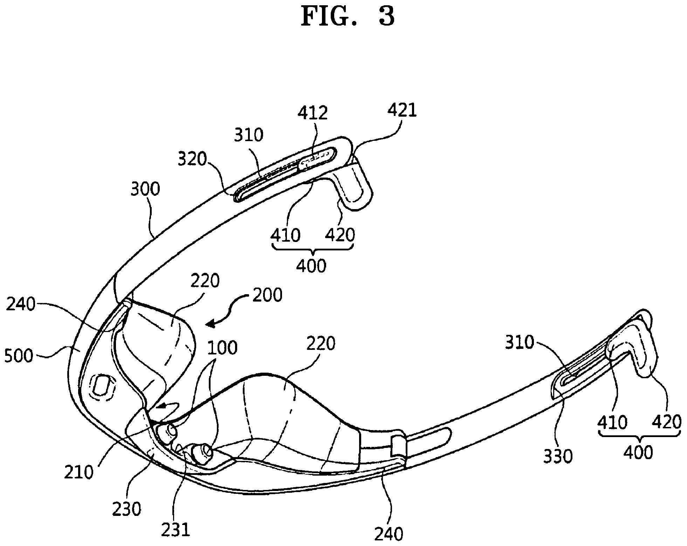

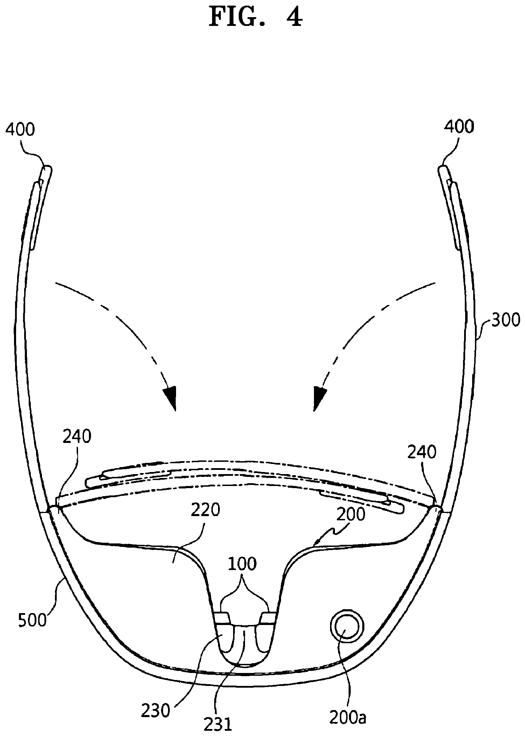

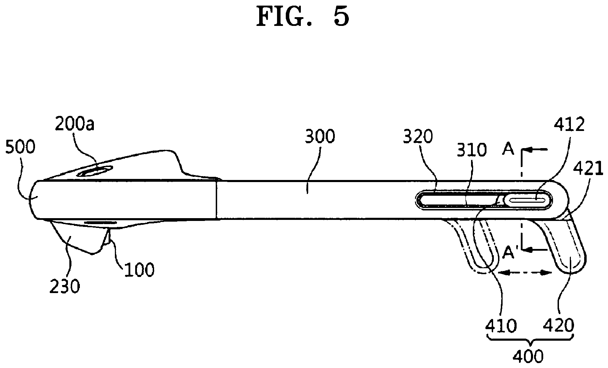

FIG. 2 is a perspective view of a face-wearing type rhinitis treatment device using light therapy, according to an embodiment of the present invention, and FIG. 3 is a bottom perspective view of the face-wearing type rhinitis treatment device using light therapy, according to an embodiment of the present invention. FIG. 4 is a top view of the face-wearing type rhinitis treatment device using light therapy, according to an embodiment of the present invention, and FIG. 5 is a lateral view of the face-wearing type rhinitis treatment device using light therapy, according to an embodiment of the present invention.

Referring to FIGS. 2 to 5, the face-wearing type rhinitis treatment device using light therapy, according to an embodiment of the present invention, will be described in detail below.

The face-wearing type rhinitis treatment device using light therapy, according to an embodiment of the present invention, includes a light irradiating unit 100 that is located below the nose of a patient and irradiates light for rhinitis treatment into the nose.

The light irradiating unit 100 is provided with a lamp for emitting light having a wavelength for treating rhinitis at an end portion.

For example, the light irradiating unit 100 irradiates red light having a wavelength from 600 nm or more to less than 700 nm. Alternatively, various modifications may be made thereto. For example, the light irradiating unit 100 may include a well-known lamp for irradiating light having a wavelength for rhinitis treatment, such as an ultraviolet ray and a laser beam.

The light irradiating unit 100 is mounted on a wearing main body 200 that may be worn on the face of a patient. Therefore, when the wearing main body 200 is worn on the face of the patient, the light irradiating unit 100 is located below the nose of the patient, such that light emitted from the lamp is irradiated into the nose, that is, into the nasal cavity.

The wearing main body 200 is worn obliquely, such that the leading end portion of the wearing main body 200 is located at an end of the nose to locate a pair of light irradiating units 100 that protrude from a rear surface of a nose supporter 230 to be apart from each other below respective nostrils and both sides of the rear end portion of the wearing main body 200 face the respective ears. Detailed descriptions thereof will be given below.

The wearing main body 200 includes a nose insertion groove 210, a face accommodating portion 220, the nose supporter 230, and a cheekbone accommodating portion 240. The wearing main body 200 may further include an on/off switch 200a according to embodiments.

The nose insertion groove 210 is formed to be gradually tapered toward the front side from an open portion of the rear side (wearing surface) of the wearing main body 200 to be stably attached on side surfaces of the nose of a patient.

Also, an inner end of the nose insertion groove 210 is formed in a round shape, such that an end portion of the nose may be stably inserted and positioned.

A lower portion of a rear side (wearing surface) of the face accommodating portion 220 is accommodated on the face of the patient, and the lower portion of the rear side accommodated on the face is not angular and is round. In other words, the face accommodating portion 220 is formed to prevent a scar on the skin of the patient when accommodated on the face of the patient and to be stably accommodated on the face of the patient by increasing a contact surface accommodated on the face.

The lower portions of both inner side surfaces of the nose insertion groove 210 of the wearing main body 200 may be accommodated on both side surfaces of the nose, and the lower portion of the leading end portion of the nose insertion groove 210 may be accommodated on and supported by the nose wing.

The boundary between the nose insertion groove 210 and the face accommodating portion 220, that is, a boundary edge connecting the rear surface of the face accommodating portion 220 and the inner surface of the nose insertion groove 210, may be round and formed as a nasal bone accommodating portion accommodated on the nasal bone.

Also, the lower portion of the nasal bone accommodating portion is formed to a round shape to be stably accommodated on the nasal bone and to increase a contact area for evenly distributing and supporting the weight of a rhinitis treatment device.

The cheekbone accommodating portion 240 is arranged to protrude from both sides of the face accommodating portion 220, such that the cheekbone accommodating portion 240 may be placed on the cheekbone of the patient. In other words, the cheekbone accommodating portion 240 protrudes from both sides of the rear side of the wearing main body 200 such that the cheekbone accommodating portion 240 may be placed on the cheekbone of the patient, and thus, the weight of the rhinitis treatment device may be further distributed.

Also, the inner surface of the cheekbone accommodating portion 240 and the corners of the boundary portion of the face accommodating portion 220 are formed in round shapes and are curved along the curve of the face of a person. The cheekbone accommodating portion 240 may be stably accommodated on the cheekbone through the curved surface.

When the wearing main body 200 is worn on the face of a patient, the nose of the patient is inserted into the nose insertion groove 210 and the lower portions of the both inner side surfaces of the nose insertion groove 210 are accommodated on the nose wing on both sides of the nose, and thus, the wearing main body 200 is supported.

Also, in the wearing main body 200, the nose insertion groove 210 is accommodated on and is supported by the nose wing on both sides of the nose, and at the same time, the rear side of the face accommodating portion 220 is accommodated on slightly protruding portions below the eye sockets.

Also, the lower portions of the nose insertion groove 210 facing each other are accommodated on the nose wing on both sides of the nose. As the rear side of the face accommodating portion 220 is accommodated on the slightly protruding portions below the eye sockets and, at the same time, the nasal bone accommodating portion formed at the boundary between the nose insertion groove 210 and the face accommodating portion 220 is accommodated on both sides of the nasal bone, the rhinitis treatment device is supported.

Also, in the wearing main body 200, the cheekbone accommodating portion 240 is accommodated on the cheekbone when the nose insertion groove 210 is accommodated on and is supported by the nose wing on both sides of the nose, the rear side of the face accommodating portion 220 is accommodated on slightly protruding portions below the eye sockets, and the nasal bone accommodating portion is accommodated on both sides of the nasal bone.

Also, the wearing main body 200 may be worn on the face of a patient while being supported at eight supporting positions including two supporting positions at which the nose insertion groove 210 is accommodated on the nose wing on both sides of the nose, two supporting positions at which the rear portion of the face accommodating portion 210 is accommodated at slightly protruding portions below the eye sockets, two supporting positions at which the nasal bone accommodating portions are accommodated on both sides of the nasal bone, and two supporting positions at which the cheekbone accommodating portion 240 is accommodated on the cheekbone at both sides of the face.

In other words, the weight generated when the rhinitis treatment device is worn on the face of the patient is distributed by the wearing main body 200 at the total eight supporting positions described above, that is, two supporting positions on the nose wing on the both sides of the nose, two supporting positions at the slightly protruding portions below the eye sockets, two supporting positions on both sides of the nasal bone, and two supporting positions above the cheekbone on both sides of the face.

Therefore, the wearing main body 200 may minimize the weight felt by the patient by distributing the weight of the rhinitis treatment device when worn on the face of the patient, and the patient may comfortably wear the rhinitis treatment device.

Also, since the wearing main body 200 is supported at the above-stated eight supporting positions, the movement of the rhinitis treatment device due to external factors, such as impact or movement of the patient, may be minimized when the rhinitis treatment device is worn on the face of the patient.

In addition, the face-wearing type rhinitis treatment device using light therapy, according to the present invention, may further include wearing support legs 300 that are arranged on both sides of the wearing main body 200 and are located on both sides of the head of the patient.

The wearing support leg 300 may be formed to have elasticity to be bent and may be worn in close contact with both sides of the head of a patient.

The wearing support leg 300 may be rotatably connected to the wearing main body 200 through a hinge and may be folded and stored when not in use.

Also, the rear end of the wearing support leg 300 may be provided with an ear hook 400 that may be hung on the ear of the patient.

The ear hook 400 protrudes downward from the rear end of the wearing support leg 300 to be hung on the ear of the patient and is located behind the ear to be hung on the ear.

The ear hook 400 helps the wearing main body 200 to be more stably worn by being hung on the ear of the patient and, at the same time, enables the weight applied as the wearing main body 200 is worn to be distributed to and supported by the ear of the patient.

The face-wearing type rhinitis treatment device using light therapy, according to the present invention, may further include an edge frame member 500 that surrounds the front surface and both side surfaces of a wearing main body and is connected to the wearing support legs 300 at both ends.

For example, the two wearing support legs 300 may be rotatably connected to both ends of the edge frame member 500 through hinges.

The edge frame member 500 is formed to a point where both ends coincide with the ends of the cheekbone accommodating portion 240, and the wearing support legs 300 are rotatably connected to both ends of the edge frame member 500 and may be folded and stored.

The wearing support legs 300 are rotatably hinge-connected to both end sides of the edge frame member 500 formed to a point coinciding with the ends of the cheekbone accommodating portion 240 and may be minimized in size when folded and stored.

The wearing support legs 300 may be unfolded around the hinges and located on both sides of the head of a patient to be located above the ears of the patient, and the ear hooks 400 are located below the ears of the patient to be hung on the ears.

Also, the wearing support legs 300 may be folded by being rotated toward each other, that is, inwardly around the hinges and stored when not in use.

The ear hooks 400 are provided to be movable in the lengthwise direction of the wearing support legs 300 to adjust the positions according to the size of the face of a patient.

In detail, the wearing support legs 300 may further include moving guide rails 310 to which the ear hooks 400 are coupled to be movable in the lengthwise direction. The ear hooks 400 are coupled to the moving guide rails 310 and may be moved along the moving guide rails 310 to adjust the positions in the lengthwise direction of the wearing support legs 300 according to the positions of ears of the patient.

The moving guide rails 310 are formed as slits with two open sides. The moving guide rails 310 have a structure that improves the wearing comfort by securing ventilation through open portions and enables smooth movement by minimizing friction.

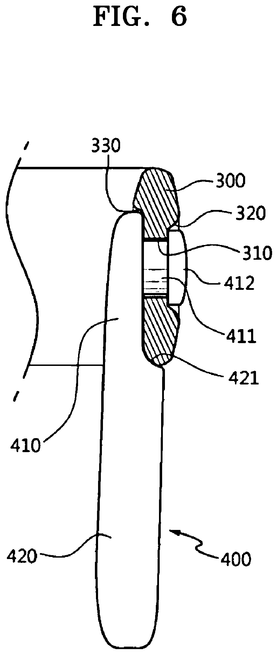

FIG. 6 is a cross-sectional view of the ear hook 400 in the face-wearing type rhinitis treatment device using light therapy, according to an embodiment the present invention. Embodiments of the ear hook 400 will be described below in more detail with reference to FIGS. 2 and 6.

The ear hook 400 includes an ear hook body 410 and an ear hook support 420.

The ear hook body 410 overlaps with the wearing support leg 300 and moves in the lengthwise direction of the wearing support leg 300. The ear hook support 420 extends from the rear end portion of the ear hook body 410 to protrude downward and is located to be hung on the ear.

The moving guide rail 310 is formed as an open slit along the lengthwise direction of the wearing support leg 300. The ear hook body 410 includes a moving body 411 and a moving guide body 412.

The moving body 411 is arranged to protrude while penetrating through and coupled with the moving guide rail 310. The moving body 411 is supported above and below the moving guide rail 310 and may move in the lengthwise direction of the moving guide rail 310. The moving guide body 412 has a size larger than that of the moving body 411 and is located at an end of the moving body 411 and the outer side surface of the wearing support leg 300, thereby preventing the moving body 411 from being separated from the moving guide rail 310. The moving guide body 412 is provided integrally with the moving body 411 to be movable while being supported by the outer side surface of the wearing support leg 300.

The wearing support leg 300 further includes a first moving rail groove 320. The moving guide rail 310 is located inside the outer side surface of the wearing support leg 300. The moving guide body 412 is inserted to the first moving rail groove 320, and thus, the first moving rail groove 320 supports the upper and low portions of the moving guide body 412 and guides movement of the moving guide body 412.

The wearing support leg 300 further includes a second moving rail groove 330. The moving guide rail 310 is located inside the inner side surface of the wearing support leg 300. The second moving rail groove 330 is formed to be opened downward and guides the movement of the moving guide body 412 by supporting the upper portion of the ear hook body 410.

Also, the ear hook support 420 includes a moving guide protrusion 421 that is formed to protrude from the ear hook body 410 to support the lower portion of the wearing support leg 300 and to move in close contact with the lower portion of the wearing support leg 300.

In other words, when the ear hook 400 is moved, the moving body 411 is moved while being supported above and below the moving guide rail 310, which has a shape of an open slit, the moving guide body 412 is moved while being supported above and below the first moving rail groove 320, the upper portion of the ear hook body 410 is moved while being supported above the second moving rail groove 330, and the lower portion of the wearing support leg 300 is moved in close contact with the upper portion of the moving guide protrusion 421. Therefore, the ear hook 400 may be stably moved.

Also, when the ear hook 400 is in a stopped state, that is, when the wearing main body 200 is worn on the face of the patient and the ear hook 400 is hung on the ear of the patient, the moving body 411 is supported above and below the moving guide rail 310, the moving guide body 412 is supported above and below the first moving rail groove 320, and the lower portion of the wearing support leg 300 is supported above the upper portion of the moving guide protrusion 421, and thus, the position of the ear hook 400 may be stably fixed.

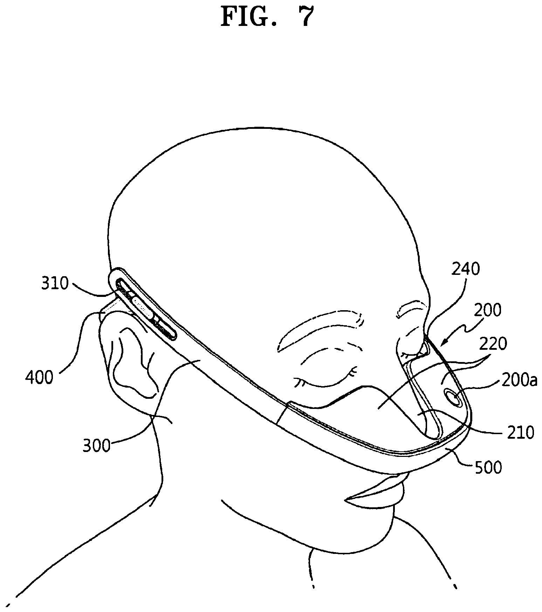

FIG. 7 is a use state diagram showing an example of wearing a face-wearing type rhinitis treatment device using light therapy, according to the present invention.

Referring to FIG. 7, the wearing main body 200 is worn obliquely, such that the leading end portion of the wearing main body 200 is located at an end of the nose to locate a pair of light irradiating units 100 that protrude from a rear surface of a nose supporter 230 to be apart from each other below respective nostrils and both sides of the rear end portion of the wearing main body 200 face the respective ears.

When the wearing main body 200 is worn, the wearing support legs 300 are located at both sides of the head of a patient, and the ear hooks 400 are hung on the ears of the patient to support the position of the wearing main body 200.

Also, the wearing main body 200 may be stably worn on the face of a patient as the wearing main body 200 is supported at eight supporting positions including two supporting positions at which the nose insertion groove 210 is accommodated on the nose wing on both sides of the nose, two supporting positions at which the rear portion of the face accommodating portion 210 is accommodated at slightly protruding portions below the eye sockets, two supporting positions at which the nasal bone accommodating portions are accommodated on both sides of the nasal bone, and two supporting positions at which the cheekbone accommodating portion 240 is accommodated on the cheekbone at both sides of the face.

The rear surface of the wearing main body 200, that is, the rear surface of the face accommodating portion 220, has a convexly protruding shape, and at least a portion of the convexly protruding portion may be accommodated above the eyelids defining the eye sockets.

At least a portion of the rear surface of the wearing main body 200 may be accommodated above the eyelids to support the weight more stably, and the rear surface of the wearing main body 200 is formed in a hemispherical convex protruding shape. Therefore, when the rear surface of the wearing main body 200 contacts the skin, the convex protruding shape evenly distributes the weight and does not cause pain even in the case of pressing portions below the eyes.

According to the present invention, the weight of a rhinitis treatment device is distributed to and supported by the nose and portions above the eyes of a patient to provide comfort while wearing the rhinitis treatment device, thereby minimizing discomfort of the patient during treatment and enabling stable long-time rhinitis treatment. As a result, satisfaction and treatment effect may be improved simultaneously.

The present invention prevents movement of the light irradiating unit for treating rhinitis caused by external factors after wearing the rhinitis treatment device, and thus the light irradiating unit for treating rhinitis may be stably maintained at a correct position under the nose to further improve treatment effect and reduce treatment time. Also, restrictions of movement of the patient after wearing the rhinitis treatment device may be minimized to minimize discomfort of the patient.

Although the present invention has been described with reference to various embodiments, the present invention is not limited to the above embodiments, and various modifications may be made thereto by one of ordinary skill in the art within the technical idea to which the present invention pertains.

## Claims

1. A face-wearing type rhinitis treatment device comprising:
a light irradiating unit configured to irradiate light for rhinitis treatment into the nose of a patient; and
a wearing main body, which is accommodated at positions spaced below the eyes of the patient and is wearable on the face of the patient,
wherein the wearing main body comprises:
a nose insertion groove, which is accommodated on the nose of the patient as the nose of the patient is inserted therein;
a face accommodating portion accommodated on the face of the patient at positions spaced below the eyes of the patient; and
a nose supporter, which is arranged at a leading end portion of the nose insertion groove for the light irradiating unit to protrude to the rear side of the nose supporter, protrudes from the lower surface of the wearing main body, and is formed to partially cover an opened lower portion of the nose insertion groove, such that an end of the nose of the patient between the nostrils is inserted therein.

2. The face-wearing type rhinitis treatment device of claim 1, wherein the wearing main body is supported as lower portions of both side surfaces of the nose insertion groove are accommodated on a nose wing on the both sides of the nose.

3. The face-wearing type rhinitis treatment device of claim 1, wherein the wearing main body further comprises
a nasal bone accommodating portion, which includes a boundary edge which connects a rear surface of the face accommodating portion to inner surfaces of the nose insertion groove and is formed round to be accommodated on the nasal bone of the patient.

4. The face-wearing type rhinitis treatment device of claim 1, wherein the wearing main body further comprises
a cheekbone accommodating portion arranged to protrude from side ends of the face accommodating portion to be placed on a cheekbone of the patient.

5. The face-wearing type rhinitis treatment device of claim 1, wherein the face accommodating portion has a rearward convex shape. and at least a portion of a rearward convex portion is accommodated above the eyelids defining the eye sockets of the patient.

6. The face-wearing type rhinitis treatment device of claim 1, wherein the nasal bone accommodating portion comprises a round lower portion.

7. The face-wearing type rhinitis treatment device of claim 1, wherein, in the wearing main body, an edge portion at the boundary between the inner surface of a cheekbone accommodating portion and the face accommodating portion is round.

8. The face-wearing type rhinitis treatment device of claim 1, further comprising:
a control unit configured to control an operation of the light irradiating unit; and
an on/off switch, which is arranged on a top surface of the wearing main body and turns the light irradiating unit on or off.

9. The face-wearing type rhinitis treatment device of claim 1, further comprising
wearing support legs, which are arranged on both sides of the wearing main body and are located on both sides of the head of the patient.

10. The face-wearing type rhinitis treatment device of claim 9, further comprising
an edge frame member, which surrounds a front surface and both side surfaces of the wearing main body and is rotatably hinge-connected to the wearing support legs at both ends.

11. The face-wearing type rhinitis treatment device of claim 9, further comprising
ear hooks, which are arranged at rear ends of the wearing support legs to be hung on the ears of the patient and are movable in a lengthwise direction of the wearing support legs.

12. The face-wearing type rhinitis treatment device of claim 11, wherein the wearing support legs further comprise
moving guide rails, which are formed as slits with opened side surfaces and connect the ear hooks to the wearing support legs to be movable in the lengthwise direction of the wearing support legs.

13. The face-wearing type rhinitis treatment device of claim 12, wherein each of the ear hooks comprises:
an ear hook body overlapping with the wearing support leg and moving in the lengthwise direction of the wearing support leg; and
an ear hook support, which extends from the rear end of the ear hook body to protrude downward and is hung on the ear of the patient.

14. The face-wearing type rhinitis treatment device of claim 13, wherein each of the ear hooks comprises:
a moving body which penetrates through and is coupled with a moving guide rail and is supported above and below the moving guide rail to move in a lengthwise direction of the moving guide rail; and
a moving guide body, which has a size larger than that of the moving body and, is located at an end of the moving body and an outer side surface of the wearing support leg to move while being supported by the outer side surface.

15. The face-wearing type rhinitis treatment device of claim 11, wherein a moving guide rail is located at the outer side surface of the wearing support leg, and the wearing support leg comprises a first moving rail groove to which the moving guide body is inserted to guide movement of the moving guide body while the first moving rail groove supports the upper and lower portions of the moving guide body.

16. The face-wearing type rhinitis treatment device of claim 11, wherein the moving guide rail is located at the inner side surface of the wearing support leg, and the wearing support leg comprises a second moving rail groove having an opened bottom to support the upper portion of the ear hook and guides movement of the moving guide body.

17. The face-wearing type rhinitis treatment device of claim 13, wherein each of the ear hook supports comprises
a moving guide protrusion, which is formed to protrude from the ear hook body and supports the lower portion of the wearing support leg to move in close contact with the wearing support leg.
